# EUROPÄISCHE PATENTANMELDUNG

(11) **EP 1 350 520 A1**
(43) Veröffentlichungstag der Anmeldung: **08.10.2003**
(21) Anmeldenummer: 03006528.8
(22) Anmeldetag: 22.03.2003
(51) Int. Cl.: A61K 38/40, A61P 7/06

(54) **Human-Transferrin zur Behandlung der Anaemia of Chronic Disease (ACD) und des funktionellen Eisenmangels**

(30) Priorität: 05.04.2002 DE 10215315
(71) Anmelder: Aventis Behring GmbH, 35039 Marburg (DE)
(72) Erfinder: Thomas, Lothar, 60489 Frankfurt (DE)

(57) **Zusammenfassung**

Es wird die Verwendung einer eine therapeutische Menge Human-Transferrin enthaltenden zur parenteralen Verabreichung geeigneten Zubereitung zur Behandlung der Anaemia of Chronic Disease (ACD) und des funktionellen Eisenmangels beschrieben. Das Human-Transferrin kann zur Behandlung anämischer Erkrankungen vorteilhaft auch in Mischung mit eisen-freiem oder eisen-beladenem Human-Transferrin und/oder Erythropoietin eingesetzt werden.

## Beschreibung

Gegenstand der Erfindung ist der Einsatz von Human-Transferrin allein oder in Kombination mit Eisen oder mit Erythropoietin oder mit Eisen und Erythropoietin zur Behandlung der Anaemia of Chronic Disease (ACD) und des funktionellen Eisenmangels.

### 1. Human-Transferrin

Human-Transferrin ist ein Eiweiß mit dem Molekulargewicht von 88 kD und wird in der Leber synthetisiert. Neben dem Proteinanteil besitzt es Kohlenhydratketten, die etwa 10% des Molekulargewichtes ausmachen. Eisen-freies Transferrin (Apotransferrin) bindet zwei Atome 3-wertiges Eisen. Normalerweise sind 16 bis 45% der A-potransferrinmoleküle mit Eisen beladen. Die Transferrin-Konzentration im Blut ist für die Eisenverteilung im Organismus verantwortlich. Von den eisen-speichernden Geweben transportiert Transferrin das Eisen in das Knochenmark zur Hämoglobinsynthese. Aus alternden Blutzellen freigesetztes Hämoglobin gibt sein Eisen wieder an Transferrin ab. Transferrin deponiert dies in eisen-speichernden Geweben und holt es dort wieder ab, wenn im Knochenmark Bedarf besteht. Transferrin ist also global gesehen für den Eisenturnover im Organismus verantwortlich. Die Konzentration im Blutserum beträgt normalerweise 2,0 bis 3,5 g/l. Bei Gesamtkörpereisenmangel bildet die Leber mehr Transferrin, die Konzentration im Serum ist erhöht, denn der Turnover des wenigen Eisens muss erhöht werden, um das Knochenmark zu versorgen. Bei der ACD ist der Eisenturnover vermindert. Ursache ist die verminderte Transferrinsynthese bei chronischen Infektionen, entzündlichen Erkrankungen und Krebsleiden, denn die Leber reguliert die Transferrinsynthese herunter, um eine verstärkte Synthese von Abwehrproteinen (Akute-Phase-Proteinen) gewährleisten zu können. Als Folge nimmt die Transferrinkonzentration im Blut ab und somit auch der Eisenturnover und die Hämoglobinbildung in den Vorläuferzellen der Erythropoiese des Knochenmarks. Auch ist bekannt, dass bei entzündlichen Erkrankungen ein verändertes Transferrin gebildet wird. So wurden bei rheumatoider Arthritis Transferrine mit stärker verzweigten Glykanseitenketten beschrieben (Feelders G.A. et al., Rheumat Int 1992; 12: 195 bis 199). Es ist anzunehmen, dass Transferrin weniger Eisen an die Transferrinrezeptoren von Hämoglobin bildenden Vorläuferzellen der Erythropoiese abgeben kann, wenn es veränderte Glykanseitenketten hat oder in verminderter Konzentration vorliegt.

### 2. Anwendungsgebiet

Etwa 10% der Bevölkerung industrialisierter Länder hat eine Anämie (Blutarmut). Häufigste Ursache der Anämie ist eine mangelnde Verfügbarkeit von Eisen, das Voraussetzung zur Bildung des roten Blutfarbstoffes Hämoglobin in den Vorläuferzellen der Erythropoiese des Knochenmarks ist. Das Hämoglobin ist der Hauptbestandteil der roten Blutzellen, die Sauerstoff von der Lunge in die Gewebe und CO₂ von den Geweben in die Lunge transportieren. Die mangelnde Verfügbarkeit von Eisen zur Hämoglobinbildung beruht entweder auf einer Verminderung des Gesamtkörpereisens oder einer Eisenverteilungsstörung. Im ersten Fall liegt eine Eisenmangelanämie vor, im zweiten ist die Menge des Gesamtkörpereisens normal oder gar erhöht, wird aber dem Knochenmark nur unzureichend zur Bildung von Hämoglobin zur Verfügung gestellt. Dieser Zustand, bei dem das Gesamtkörpereisen adäquat ist, aber ein Mangel besteht, es entsprechend den Erfordernissen für die Hämoglobinbildung zur Verfügung zu stellen, wird als funktioneller Eisenmangel bezeichnet. Das ist der Fall bei allen Anämien, die im Rahmen chronischer Infektionen, entzündlicher Erkrankungen und Krebsleiden auftreten (Means RT, Krantz SB, Blood 1992; 80: 1639 bis 1647). Auch ist das der Fall bei allen Anämien mit inadäquat niedriger Erythropoietinbildung, bei denen es nach Stimulierung der roten Blutzellbildung durch rekombinantes humanes Erythropoietin (r-HuEPO) zu einer mangelnden Versorgung des Knochenmarks mit Eisen kommt. Das ist klassischerweise die Situation bei der Behandlung von Dialysepatienten im Endstadium der chronischen Niereninsuffizienz mit r-HuEPO. Denn die therapeutische Gabe von r-HuEPO kann die rote Blutzellbildung in einem solchen Ausmaß stimulieren, dass der Bedarf des proliferierenden Knochenmarks an Eisen die Bereitstellung weit übertreffen kann (Cavill I, Blood 1993; 83: 1377).

### 3. Anwendung von Human-Transferrin

Human-Transferrin enthaltende pharmazeutische Zubereitungen haben bereits therapeutische Anwendungen gefunden. So ist es aus den US-Patenten 6 251 860 und 6 326 473 bekannt, Apotransferrin, also die eisen-freie Form des Transferrins, enthaltende Präparationen einzusetzen, um Nicht-Transferrin-gebundenes Eisen (NTBI) zu binden, welches unter zytotoxischer Therapie von Krebspatienten auftritt und zu Gewebeschädigungen führen kann. Die Anwendung von eisen-freiem oder eisen-haltigem Human-Transferrin zur Behandlung von Anämien, insbesondere der Anaemia of Chronic Disease (ACD) und des funktionellen Eisenmangels wird dort allerdings nicht erwogen oder nahegelegt.

Die ausreichende Versorgung des Knochenmarkes mit Eisen hat in den letzten Jahren eine immer größer werdende Bedeutung erhalten, weil durch den zunehmenden Einsatz von r-HuEPO bei an Anämie leidenden Patienten und die dadurch stimulierte Hämoglobin-Bildung eine immer stärker werdende Verarmung des Knochenmarks an Eisen beobachtet wird, die mit den bisher bekannten Eisensubstitutionstherapien nicht mehr ausreichend zu beherrschen ist. Die zusätzliche Gabe von Eisen bei der rHuEPO-Therapie führt in diesen Fällen zur Vermehrung des Gesamtkörpereisens und zur Schädigung von Organen durch oxidativen Stress.

Es wurde nun gefunden, dass überraschenderweise diese Probleme durch die Verabreichung von Human-Transferrin in einer äußerst befriedigenden Weise gelöst werden können.

Gegenstand der Erfindung ist deshalb die Verwendung einer eine therapeutisch wirksame Menge Human-Transferrin enthaltenden, zur parenteralen Verabreichung geeigneten Zubereitung zur Behandlung der Anaemia of Chronic Disease (ACD) und des funktionellen Eisenmangels. Diese Zubereitung kann eisen-freies oder eisen-beladenes Human-Transferrin in Abhängigkeit vom Eisenstatus des Patienten enthalten.

Besonders gute Therapieerfolge bei der Behandlung anämischer Erkrankungen werden mit einem Kombinationspräparat erzielt, das voneinander getrennte oder in Mischung vorliegende, zur parenteralen Verabreichung geeignete Zubereitungen therapeutischer Mengen eines
a) eisen-freien oder eisen-beladenen Human-Transferrins enthält und/oder
b) Erythropoietins enthält.

Das hierbei eingesetzte Human-Transferrin kann aus Serum oder Plasma gewonnen. Ebenso kann ein rekombinantes Transferrin eingesetzt werden, wobei die korrekte Glycosylierung wichtig ist. Ein zur Gewinnung von eisen-freiem Human-Transferrin gut geeignetes Verfahren ist aus der europäischen Patentanmeldung 0 490 384 bekannt. Besonders bevorzugt ist allerdings die Anwendung eines mit Eisen gesättigten Human-Transferrins.

Das für die erfindungsgemäßen pharmazeutischen Zubereitungen eingesetzte Erythropoietin wird nach rekombinanten Methoden hergestellt.

Es konnte nun eindeutig gezeigt werden, dass die Gabe von gereinigtem Human-Transferrin einen positiven Effekt bei der Behandlung der ACD und bei funktionellem Eisenmangel hat. Innerhalb von 5 Tagen kommt es zum Anstieg des Hämoglöbins in den Vorläuferzellen der Erythropoiese. Bei ACD ist die Transferrinkonzentration im Serum vermindert (<2g/l) und das Transferrin ist strukturell an den Kohlehydratseitenketten verändert. Zusätzlich ist die Sättigung des Transferrins mit Eisen vermindert (Transferrinsättigung < 16%). Bei funktionellem Eisenmangel ist der Eisenturnover unzureichend, denn die proliferierende Erythropoiese wird nicht adäquat mit Eisen durch eisen-beladenes Transferrin versorgt.

Überraschenderweise wird nun durch die intravenöse Gabe von Apotransferrin, Transferrin oder eisen-gesättigtem Transferrin die Eisenversorgung der Erythropoiese verbessert und somit eine Anämie korrigiert. Die Gabe der jeweiligen Zubereitung richtet sich dabei nach dem Gesamtkörpereisenstatus des Patienten. Klinische und labordiagnostische Beobachtungen bei Patienten mit ACD und funktionellem Eisenmangel haben gezeigt, dass die Gabe von Fresh Frozen Plasma, das Transferrin mit normaler Eisensättigung enthält, zuerst den Hämoglobingehalt in Retikulozyten steigert und dann zu einem Anstieg des Hämoglobinwertes im Blut führt.

Die Verabreichung der erfindungsgemäßen pharmazeutischen Transferrinzubereitungen sollte als intravenöse Injektion oder als Infusion erfolgen. Die wirksame Dosierung variiert von 50 bis 250 mg/kg und Tag und liegt bei der ACD eher an der unteren und dem funktionellem Eisenmangel eher an der oberen Grenze. Bei Gabe von eisen-gesättigtem Transferrin anstatt der Gabe von intravenösen Eisen in Kombination mit Erythropoietin konnte beobachtet werden, dass die bekannten Nebenwirkungen von intravenösen Eisentherapien nicht auftreten. Auch treten toxische Konzentrationen von Nicht-Transferrin-gebundenem Eisen (NTBI), die bei der oralen Eisentherapie üblich sind, nicht auf. Der durch Nicht-Transferrin-gebundenes Eisen verursachte oxidative Stress und die damit verbundenen Gewebe- und Organschäden (Knight J.A. ed. AACC Press 1999; 1 bis 392) können durch die Gabe von Transferrin-gebundenem Eisen verhindert werden.

## Patentansprüche

1. Verwendung einer eine therapeutisch wirksame Menge Human-Transferrin enthaltenden, zur parenteralen Verabreichung geeigneten Zubereitung zur Behandlung der Anaemia of Chronic Disease (ACD) und des funktionellen Eisenmangels.

2. Verwendung nach Anspruch 1, **dadurch gekennzeichnet, dass** die Zubereitung eisenfreies oder eisenbeladenes Human-Transferrin enthält.

3. Pharmazeutische Zubereitung zur Behandlung anämischer Erkrankungen, **dadurch gekennzeichnet, dass** es voneinander getrennte oder in Mischung vorliegende, zur parenteralen Verabreichung geeignete Zubereitungen therapeutisch wirksamer Mengen eines
a) eisenfreien oder eisenbeladenen Human-Transferrins und/oder
b) Erythropoietins
enthält.

4. Pharmazeutische Zubereitung nach den Ansprüchen 1 - 3, **dadurch ge kennzeichnet, dass** aus Serum oder Plasma gewonnenes Human-Transferrin ein gesetzt wird.

5. Pharmazeutische Zubereitung nach den Ansprüchen 1 bis 3, **dadurch gekennzeichnet, dass** bevorzugt rekombinant hergestelltes Human-Transferrin eingesetzt wird.

6. Pharmazeutische Zubereitung nach den Ansprüchen 1 bis 3, **dadurch gekennzeichnet, dass** rekombinante Fragmente von Human-Transferrin oder Fragmente von rekombinant hergestelltem Human-Transferrin oder Fragmente von aus Plasma oder Serum hergestelltem Human-Transferrin eingesetzt werden.

7. Pharmazeutische Zubereitung nach den Ansprüchen 3 und 4, **dadurch gekennzeichnet, dass** ein mit Eisen gesättigtes Human-Transferrin eingesetzt wird.

8. Pharmazeutische Zubereitung nach den Ansprüchen 1 bis 3, **dadurch gekennzeichnet, dass** ein rekombinantes Erythropoietin eingesetzt wird.

9. Verwendung der Pharmazeutische Zubereitung nach den Ansprüchen 3 bis 8, **dadurch gekennzeichnet, dass** es zur Behandlung der Anaemia of Chronic Disease (ACD) und des funktionellen Eisenmangels eingesetzt wird.
